Europäisches Patentamt

European Patent Office

Office européen des brevets

(11) Publication number: **0 454 738 B1**

# EUROPEAN PATENT SPECIFICATION

(45) Date of publication of patent specification: **26.04.95** (51) Int. Cl.⁶: **A61M 1/00**, A61M 5/168

(21) Application number: **90902176.8**

(22) Date of filing: **19.01.90**

(86) International application number:
**PCT/DK90/00019**

(87) International publication number:
**WO 90/07942 (26.07.90 90/17)**

The file contains technical information submitted
after the application was filed and not included in
this specification

(54) **A METHOD OF CONTINUOUS MONITORING OF THE OPERATION OF A DELIVERY SYSTEM AND A DEVICE FOR CARRYING OUT THIS METHOD.**

(30) Priority: **20.01.89 PL 277308**

(43) Date of publication of application:
**06.11.91 Bulletin 91/45**

(45) Publication of the grant of the patent:
**26.04.95 Bulletin 95/17**

(84) Designated Contracting States:
**AT BE CH DE DK ES FR GB IT LI LU NL SE**

(56) References cited:
**EP-A- 0 040 592**
**EP-A- 0 291 727**
**EP-A- 0 296 124**
**WO-A-84/01719**
**US-A- 4 762 518**

(73) Proprietor: **INSTITUTE OF BIOCYBERNETICS AND BIOMEDICAL ENGINEERING P.A.S.**
**ul. Krajowej Rady Narodowej 55**

**00-818 Warsaw (PL) (PL)**

Proprietor: **NOVO NORDISK A/S**
**Novo Allé**
**DK-2880 Bagsvaerd (DK)**

(72) Inventor: **WOJCICKI, Jan**
**Lipska 40 apt. 4**
**03-908 Warsaw (PL)**
Inventor: **LILPOP, Boguslaw**
**Szopena 6 apt. 25**
**05-800 Pruszk w (PL)**
Inventor: **ZIEMBICKI, Marek**
**Mandarynki 2 apt. 5**
**Warsaw (PL)**
Inventor: **BIELAWSKI, Stanislaw**
**Polna 54 apt. 9**
**Warsaw (PL)**

EP 0 454 738 B1

(74) Representative: **Hansen, Einar Tronier et al
c/o Novo Nordisk A/S,
Patent Department,
Novo Allé
DK-2880 Bagsvaerd (DK)**

## Description

The present invention relates to a method of continuous monitoring of the operation of a delivery system, in particular for use in insulin administration in the treatment of type-I diabetes, a device for carrying out this method, and the use of this device.

Delivery systems including the device of the present invention are suitable for use not only for medical treatment purposes but it may as well be used as a batch feeder in analytical laboratories, in the chemical and pharmaceutical industries and so on.

There exists a number of different constructional solutions of problems of portable and implantable insulin delivery systems. For instance, a system may consist of: A power supply unit, a drug reservoir, a control system, a volumetric pump with a measuring element and measurement system.

The insulin delivery system usually enables insulin infusion in two forms: (1) the basic infusions aimed to cover the daily requirement of insulin of the body and (2) additional or supplementary infusions meant to compensate the meals - i. e. the bolus infusions - or adjusting the volume of infusion according to increased physical activity or to an increased demand as caused by the so-called dawn phenomenon (The dawn phenomenon is the blood glucose rise taking place between 3 a. m. and 8 a. m. in some individuals.).

Safe operation of an insulin delivery system is a fundamental requirement as a malfunction of a device causing either an overdose of the hormone or a lack of infusion of the hormone constitutes a serious hazard to the life of the patient.

In order to minimize the risk caused by the malfunction in two examples of the best solutions until now, viz. the American delivery systems Betatron I and II (manufactured by Cardiac Pacemakers Inc.), the following alarm-states are signalled: Supply voltage drop, empty drug reservoir, blockage of the outlet catheter, microcomputer break-down, failure of the internal memory of the system, a too high frequency of the pump operation, motor break-down, dose of insulin in excess of the established daily dose of insulin and erroneous initial data input (improper programming of the infusion). The Betatron I and II delivery systems are described in some detail in Health Devices, November 1987, "Ambulatory Insulin Infusion Pumps", pp. 351-376 (in which nine ambulatory insulin infusion pumps are evaluated).

In another construction, viz. MRS-1 (manufactured by Disetronic AG/Ltd.) the following features are controlled: Electronic systems, supply battery, drug level in the reservoir, outlet catheter patency and excess doses of insulin administered in the form of boluses.

In the solutions mentioned above alarm states such as empty drug reservoir, a too high frequency of the operation of the pump or a dose of insulin in excess of the daily allowable insulin dose are determined by counting control impulses fed to the pump. Hence, such a monitoring method provides merely an indirect monitoring of the correctness of insulin infusion. This is also true of other alarm states. For instance, monitoring of the operation of the pump or of the supply battery is carried out by measuring the mechanical movement and by measuring the supply voltage level respectively.

Another issue or problem is connected with the monitoring of the threshold pressure in the outlet catheter of insulin delivery systems (monitoring with a view to "catheter plugging"). A solution to this issue or problem as developed by Siemens is described in: K. Prestele and M. Frentzki, "State of Development of Program-Controlled Implantable Insulin Delivery Systems" in "Artifcial Systems for Insulin Delivery" edited by P. Brunetti et al., 1983, Raven Press, New York, pp. 141-153, in particular pp. 149-150. According to this method the pressure measurement is performed as an indirect non-quantitative check of the insulin flow through the pump outlet. In the discussed solution the system for the measurement of the pressure consists of a non-conducting cylinder having a diameter equal to the inner diameter of the catheter, two electrodes and a unit for measuring the conductivity in the circuit: Electrode - catheter section containing the cylinder - electrode. Under conditions of normal operation of the pump the overpressure generated causes an increase of both the diameter of the catheter and of the flow of the insulin (which is a conductor) around the cylinder. A blockage of the catheter produces a much stronger signal than the signal received during proper operation. If the pressure threshold value is exceeded by 1 bar the alarm is switched on.

The above examples of the best solutions until now of the problems of checking the proper operation of insulin delivery systems control or monitor the operation of particular units or elements of the systems in ways that allow only indirect monitoring of the correctness of the insulin infusion. The control or monitoring of the number of revolutions of the motor per unit of time or the check of sequences of impulses fed to the pump can by no means lead to a determination of whether the insulin infusion into the body of a patient is proper since there can always be a broken catheter or the pump can be feeding air instead of the drug and so on.

The above mentioned examples have one thing in common: They are all examples of "open-loop" methods of continuous monitoring of the operation of portable insulin delivery systems, pumps for short,

3

and/or "open-loop" type control or monitoring devices for use in carrying out such methods.

"EP 40 592 describes an infusion pump for intravenous or intra-arterial infusion of liquid medicines by which pump an alarm signal is generated when certain parameters are beyond set limits. The parameters comprise the blood pressure, the pressure in the infusion catheter, and the dosage actually delivered. These parameters are each separately monitored by transducers giving off their measuring signals to a control circuit."

"A sensor which may be mounted in a catheter to measure the pressure in this catheter is described in EP 115 548."

Having regard to the above mentioned prior art, and the problems mentioned above, it is the object of the present invention to provide a method of continuous monitoring of a delivery system comprising a volumetric pump, an infusion control unit, an electronic measuring unit and an outlet catheter provided with a pressure sensor in the following referred to as a sensor, and by means of which method at least some of the problems mentioned above will be solved and a device for carrying out this method and by means of which device at least some of the problems mentioned above will be solved.

The stated object of the present invention is achieved by a method as characterized in Claim 1.

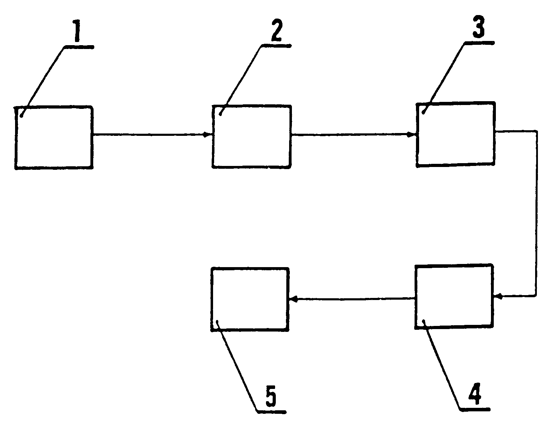

It has been found - as mentioned below in the description of how the method of the present invention works, this description being given in relation to the drawing - that between the amplitude of the measurement signal - calculated as the difference between the peak value and zero level - on one hand and the pressure of a single pump delivery on the other hand there exists a dependence of some sort which dependence of some sort may be a linear dependence.

Through the control or monitoring of the proper value of the electric signal amplitude from the measuring element every single delivery of the pump is monitored.

The signal from the measuring element is used simultaneously for monitoring the resistance to the flow of the liquid from the drug reservoir to the pump and from the pump to the patient as well as for checking the supply voltage. This is possible thanks to changes in the signal amplitude occuring whenever any of the above parameters change their value.

Compared to the prior art this is worthy of note since it means that the method of the present invention can be a method of the "closed-loop" type in contrast to the methods of the prior art mentioned above that are of the "open-loop" type.

It is further worthy of note that in turn this means that in the method of the present invention there is provided a feedback that can be used to control the insulin infusions.

The method of monitoring of the present invention is not related to the performance of any particular element of one or another insulin delivery system but solely to the actual efficiency of the insulin delivery system as measured in the catheter.

It is an advantage in connection with the method of the present invention that the actual delivery of the pump is measured at the outlet of the catheter by measuring the amplitude of the electric signal coming from the measuring element or sensor which signal depends on the pressure and/or the volume of the delivery of the pump and which signal is then compared to preset reference values.

It is another advantage in connection with the method of the present invention that the signal from the measuring element or sensor is transmitted via a matching system to a measurement system and also that the measurement results obtained in the measurement system are transmitted to an analog-to-digital converter and from this analog-to-digital converter to an analysing system before being analysed in this analysing system.

The stated object of the present invention is further achieved by a device as defined in Claim 13.

It has been found that between the amplitude of the measurement signal - calculated as the difference between the peak value and zero level - on one hand and the pressure and/or the volume of a single pump delivery on the other hand there exists a dependence of some sort which dependence of some sort may be a linear dependence.

Through the control or monitoring of the proper value of the electric signal amplitude from the measurement element or sensor every single delivery of the pump is monitored.

The signal from the measuring element is used simultaneously for monitoring the resistance to the liquid flow at drug reservoir to pump and pump to patient as well as for checking the supply voltage. This is possible thanks to changes in the signal amplitude occuring whenever any of the above parameters change their value.

It is further worthy of note that in turn this means that by means of the device of the present invention there is provided a means for feedback that can be used to control the insulin infusions.

The device of the present invention for continuous monitoring of a delivery system consisting of a volumetric pump, an infusion control unit, an electronic measurement unit and an outlet catheter provided with a measuring element and for use in carrying out the method of the present invention is not related to any particular element of one or another insulin delivery system but solely to the actual insulin delivery system comprising a measuring element in the catheter.

It is an advantage in connection with the device of the present invention that the device comprises a measuring element or sensor placed in the catheter and a system for comparing the signal coming from the measurement element or sensor to preset reference values.

It is another advantage in connection with the device of the present invention that the device comprises a measuring element or sensor placed in the catheter, a matching system, a measurement system, an analog-to-digital converter and an analysing system.

Below the invention will be explained in some detail while having reference to an example of an embodiment of the method of the present invention in which the example of an embodiment of the device of the the present invention shown in the drawing is being utilized. It should be noted that this embodiment of the method of the present invention and that this embodiment of the device of the present invention are given solely as examples and should in no way be considered as limiting the invention in one or more respects.

The drawing shows a block diagram of a delivery system having a volumetric pump and controlled by the delivery of the pump.

In the drawing a measuring element or sensor is provided in the catheter. The signal from this measuring element 1 or sensor 1 is transmitted via a matching system 2 to a measurement system 3. The measurement results obtained in 3 are transmitted to an analog-to-digital converter 4 before being analysed in a system 5.

When the embodiment of the method of the present invention illustrated in the drawing is being used while utilizing the embodiment of the device of the present invention illustrated in the drawing displacement of the fluid by the pump causes the occurence of an overpressure in the pump section which causes the measuring element 1 or sensor 1 to give the signal, the amplitude of which depends on the pressure of the pump delivery. The overpressure decays when the single cycle of the pump work is completed. The courses of pressure measured by the measuring element 1 in the intervals between the consecutive cycles of the operation of the pump as well as during each cycle of the operation of the pump are converted to electric analog signals. These signals are transmitted via the matching system 2 to the measurement system 3 where the zero level of the signal coming from the sensor 1 (pump on idle) as well as its peak value (pump operates) are measured. The measured parameters are transmitted to the analog-to-digital converter 4 and are next analysed in the system 5.

It has been found that between the amplitude of the measurement signal - calculated as the difference between the peak value and zero level - on one hand and the volume of a single pump delivery on the other hand there exists a dependence of some sort which dependence of some sort may be a linear dependence. Therefore in the discussed solution of the problems of delivery systems the continuous quantitative control or monitoring of the actual delivery of the pump is carried out. Monitoring of the delivery operation of the system consists in continuous comparison of the measured values to the reference values recorded (1) in the memory of the system for comparing the signal coming from the measuring element or sensor to preset reference values or more specifically (2) in the memory of the analysing system 5 and in - if there occurs a discrepency - setting on the alarm.

The measurement signal generated by the measuring element also works as a very sensitive indicator of several malfunctions that may occur during the operation of the delivery system. This concerns: Supply voltage drop, positive or negative external pressure influence, outlet catheter blockage, breakage of the catheter or disconnection of the catheter from the patient, blockage of the connector between the drug reservoir and the pump as well as appearance of an air bubble in the pump.

The occurence of any of the disturbances mentioned above causes a decrease or increase in the magnitude of the signal - a drop or rise of the signal - coming from the sensor 1. Consequently, passing the upper or lower values of the settled or preset limits for amplitude, peak value or zero level switches the device to the alarm states.

An example of the schematic description of the decisive signal changes is given in the table to follow.

## TABLE, PART ONE

| Disturbances | Symptoms caused by the disturbances | Possible alarm states |
|---|---|---|
| Battery voltage drop | ↓ (Peak value gradual drop) | I |
| Air bubble inside pump mechanism | ↓ (Peak value rapid drop) | II |
| Blockage of the connector between reservoir and pump | ↓ (Peak value rapidly accelerating drop) | II |
| Increase in the stroke volume | ↑ (Peak value rapid rise) | III |
| Decrease in the stroke volume | ↓ (Peak value rapid drop) | I |
| Influence of an external negative hydrostatic pressure | ↓ (Zero level rapid drop) | IV |
| Influence of an external positive hydrostatic pressure | ↑ (Zero level rapid rise) | V |

TABLE, PART TWO

| Disturbances | Symptoms caused by the disturbances | Possible alarm states |
|---|---|---|
| Blockage of the catheter | ↑ (zero level rapidly increasing rise) | VI |
| Catheter cracks | ↑ (Zero level rapid rise) | V |
| Disconnection of the catheter from the body | ↑ (Zero level rapid rise) | V |

↑ ↓  - direction of changes

The alarm states indicated by roman numerals are not exclusive.

The occurence of any of the disturbances mentioned above changing the signal from the measuring element, thus in the case of supply battery voltage drop, change of a positive external pressure, blockage of the outlet catheter or of the connector between the drug reservoir and the pump or an air bubble in the liquid - causes a rapid drop of the amplitude of the signal coming from the sensor 1 or - in the case of change in the negative external pressure, breakage of the catheter or the disconnection of the catheter from the patient - its rise. Consequently passing the upper or lower values of the settled or preset limit switches the device into one or more of the alarm states.

Thus, the measured signal coming from the sensor 1 can be utilized for simultaneous monitoring of the operation of the pump and of the patency of the junctions from drug reservoir to the pump and from the pump to the patient as well as for checking the supply voltage of the system.

Although the method and the device and the use according to the invention were developed primarily with a view to the administration of insulin to diabetic patients, the method and the device will also be useful in connection with the administration of other dissolved drugs which advantageously can be administered over a prolonged period of time. Examples of such drugs are heparin, oxytocin, human growth hormone and cancer chemotherapeutics.

**Claims**

1. A method of continuous monitoring of a delivery system, in particular for use in insulin administration, comprising a volumetric pump made to deliver a preset amount of liquid by each cycle of the pump, an infusion control unit, an electronic measuring unit and an outlet catheter provided with a measuring element, **characterized** in that during every single cycle of the pump the actual pressure of delivery is measured, preferably continuously, in the catheter and is compared with recorded values, and deviations of the measured values from the recorded values are interpreted as indicating malfunction.

2. A method according to claim 1, **characterized** in that the pressures of delivery to be compared with recorded values are the zero level (pump not activated) and the peak pressure value (during the pump stroke).

3. A method according to claim 1 or 2, **characterized** in that the results of the comparisons are interpreted to define whether the state of the device is normal with respect to the following sources of error: 1) Supply voltage; 2) Decrease in stroke volume (Alarm State I); 3) Air bubble inside pump; 4) Blockage of the connector between reservoir and pump (Alarm state II); 5) Increase in the stroke volume (Alarm State III); 6) Influence of an external negative hydrostatic pressure (Alarm State IV); 7) Influence of an external positive hydrostatic pressure; 8) Catheter broken; 9) Catheter disconnected from the body of the patient (Alarm State V); and 10) Blockage of the catheter between the measuring element and the outlet (Alarm State VI).

4. A method according to claim 3, **characterized** in that a gradual drop of the peak value is interpreted as indicating a battery voltage drop.

5. A method according to claim 3, **characterized** in that a rapid drop of the peak value is interpreted as indicating an air bubble inside the pump mechanism.

6. A method according to claim 3, **characterized** in that a rapidly accelerating drop of the peak value is interpreted as indicating a blockage of the connection between reservoir and pump.

7. A method according to claim 3, **characterized** in that a rapid rise of the peak value is interpreted as indicating an increase in the amount of liquid delivered during a cycle of the pump.

8. A method according to claim 3, **characterized** in that a rapid drop of the peak value is interpreted as indicating a decrease in the amount of liquid delivered during a cycle of the pump.

9. A method according to claim 3, **characterized** in that a rapid drop of the zero level is interpreted as indicating the influence of an external negative hydrostatic pressure.

10. A method according to claim 3, **characterized** in that a rapid rise of the zero level is interpreted as indicating the influence of an external positive hydrostatic pressure.

11. A method according to claim 3, **characterized** in that a rapidly increasing rise of the zero level is interpreted as indicating a blockage of the catheter.

12. A method according to claim 3, **characterized** in that a rapid rise in zero level is interpreted as indicating catheter cracks or disconnection of the catheter from the body.

13. A device for continuous monitoring of a delivery system, in particular for use in insulin administration, consisting of a volumetric pump made to deliver a preset amount of liquid by each cycle of the pump, an infusion control unit, an electronic measuring unit and an outlet catheter provided with a measuring element (1) according to any of the claims 1 -12, **characterized** in that it comprises a measuring element (1) generating a signal reflecting the actual pressure in the catheter, and an analyzing system (5) which compares the measured values with values recorded in a memory of the analyzing system (5) and gives off a signal if the measured values deviates from the values recorded in the memory.

**Patentansprüche**

1. Verfahren zum kontinuierlichen Überwachen eines Ausgabesystems, insbesondere zur Verwendung bei der Verabreichung von Insulin, das eine volumetrische Pumpe, die zum Abgeben einer voreingestellten Flüssigkeitsmenge mit jedem Zyklus der Pumpe dient, eine Infusionssteuereinheit, eine elektronische Meßeinheit und einen Auslaßkatheter umfaßt, der mit einem Meßelement versehen ist, dadurch gekennzeichnet, daß während jedes einzelnen Zyklus der Pumpe der tatsächliche Ausgabedruck, vorzugsweise kontinuierlich, in dem Katheter gemessen und mit aufgezeichneten Werten verglichen wird und Abweichungen der gemessenen Werte von den aufgezeichneten Werten als Hinweis auf eine Funktionsstörung interpretiert werden.

2. Verfahren nach Anspruch 1, dadurch gekennzeichnet, daß die Ausgabedrücke, die mit aufgezeichneten Werten zu vergleichen sind, der Nullpunktdruckwert (Pumpe nicht betrieben) und der Spitzendruckwert (während des Pumpenhubs) sind.

3. Verfahren nach Anspruch 1 oder 2, dadurch gekennzeichnet, daß die Ergebnisse der Vergleiche interpretiert werden, um zu bestimmen, ob der Zustand der Vorrichtung normal ist in Bezug auf die folgenden Fehlerquellen: 1) Versorgungsspannung; 2) Abnahme des Hubvolumens (Alarmzustand I); 3) Luftblasen innerhalb der Pumpe; 4) Verstopfung des Verbinders zwischen Reservoir und Pumpe (Alarmzustand II); 5) Anstieg des Hubvolumens (Alarmzustand III); 6) Einfluß eines äußeren hydrostatischen Unterdrucks (Alarmzustand IV); 7) Einfluß eines äußeren hydrostatischen Überdrucks; 8) Katheter zerstört; 9) Katheter vom Körper des Patienten des Patienten gelöst (Alarmzustand V); und 10) Verstopfung des Katheters zwischen dem Meßelement und dem Auslaß (Alarmzustand VI).

4. Verfahren nach Anspruch 3, dadurch gekennzeichnet, daß eine allmähliche Abnahme des Spitzenwerts als ein Hinweis auf eine Batteriespannungsabnahme interpretiert wird.

5. Verfahren nach Anspruch 3, dadurch gekennzeichnet, daß eine schnelle Abnahme des Spitzenwerts als ein Hinweis auf Luftblasen innerhalb des Pumpenmechanismus interpretiert wird.

6. Verfahren nach Anspruch 3, dadurch gekennzeichnet, daß eine sich schnell beschleunigende Abnahme des Spitzenwerts als ein Hinweis auf eine Verstopfung der Verbindung zwischen Reservoir und Pumpe interpretiert wird.

7. Verfahren nach Anspruch 3, dadurch gekennzeichnet, daß ein schneller Anstieg des Spitzenwerts als ein Hinweis auf einen Anstieg der Flüssigkeitsmenge, die während eines Zyklus der Pumpe abgegeben wird, interpretiert wird.

8. Verfahren nach Anspruch 3, dadurch gekennzeichnet, daß eine schnelle Abnahme des Spitzenwerts als ein Hinweis auf eine Abnahme der Flüssigkeitsmenge, die während eines Zyklus der Pumpe abgegeben wird, interpretiert wird.

9. Verfahren nach Anspruch 3, dadurch gekennzeichnet, daß eine schnelle Abnahme des Nullpunkts als Hinweis auf den Einfluß eines äußeren hydrostatischen Unterdrucks interpretiert wird.

10. Verfahren nach Anspruch 3, dadurch gekennzeichnet, daß ein schneller Anstieg des Nullpunkts als ein Hinweis auf den Einfluß eines äußeren hydrostatischen Überdrucks interpretiert wird.

11. Verfahren nach Anspruch 3, dadurch gekennzeichnet, daß ein schnell zunehmender Anstieg des Nullpunkts als ein Hinweis auf eine Verstopfung des Katheters interpretiert wird.

12. Verfahren nach Anspruch 3, dadurch gekennzeichnet, daß ein schneller Anstieg des Nullpunkts als ein Hinweis auf Katheterrisse oder ein Loslösen des Katheters von dem Körper interpretiert wird.

13. Vorrichtung zum kontinuierlichen Überwachen eines Ausgabesystems, insbesondere zur Verwendung bei der Verabreichung von Insulin, die aus einer volumetrischen Pumpe, die zum Abgeben einer voreingestellten Flüssigkeitsmenge mit jedem Zyklus der Pumpe dient, einer Infusionssteuereinheit, einer elektronischen Meßeinheit und einem Auslaßkatheter besteht, der mit einem Meßelement (1) gemäß einem der Ansprüche 1 bis 12 versehen ist, dadurch gekennzeichnet, daß sie ein Element (1), das ein Signal erzeugt, das den tatsächlichen Druck in dem Katheter wiedergibt, und ein Analysiersystem (5) umfaßt, das die gemessenen Werte mit in einem Speicher des Analysiersystems (5) aufgezeichneten Werten vergleicht und ein Signal aussendet, wenn die gemessenen Werte von den in dem Speicher aufgezeichneten Werte abweichen.

**Revendications**

1. Procédé de surveillance en continu d'un système d'alimentation utilisé en particulier dans le cadre de l'administration d'insuline, comprenant une pompe volumétrique réalisée pour fournir une quantité prédéterminée de liquide pour chaque cycle de la pompe, une unité de commande de perfusion, une unité de mesure électronique et un cathéter de sortie muni d'un élément de mesure, caractérisé en ce que pendant chaque cycle individuel de la pompe, la pression réelle de l'administration est mesurée, de préférence en continu, dans le cathéter et en ce qu'elle est comparée aux valeurs enregistrées et les écarts entre les valeurs mesurées et les valeurs enregistrées sont interprétés comme indiquant un mauvais fonctionnement.

2. Procédé selon la revendication 1, caractérisé en ce que les pressions d'alimentation à comparer avec les valeurs enregistrées sont le niveau zéro (pompe non activée) et la valeur de pression de pointe (pendant la course de la pompe).

3. Procédé selon la revendication 1 ou 2, caractérisé en ce que les résultats des comparaisons sont interprétés pour définir si l'état du dispositif est normal par rapport aux sources suivantes d'erreur : 1) tension électrique d'alimentation; 2) diminution du volume de course de la pompe (alarme état I); 3) bulle d'air à l'intérieur de la pompe; 4) obstruction de la liaison entre le réservoir et la pompe (alarme état II); 5) augmentation du volume de la course de la pompe (alarme état III); 6) influence d'une pression hydrostatique négative externe (alarme état IV); 7) influence d'une pression hydrostatique positive externe; 8) cathéter rompu; 9) cathéter débranché du corps du patient (alarme état V); et 10) obturation du cathéter entre l'élément de mesure et la sortie (alarme état VI).

4. Procédé selon la revendication 3, caractérisé en ce qu'une chute progressive de la valeur de pic est interprétée comme indiquant une chute de tension électrique de la batterie.

5. Procédé selon la revendication 3, caractérisé en ce qu'une chute rapide de la valeur de pic est interprétée comme indiquant la présence d'une bulle d'air à l'intérieur du mécanisme de la pompe.

6. Procédé selon la revendication 3, caractérisé en ce qu'une chute à accélération rapide de la valeur de pic est interprétée comme indiquant une obturation de la liaison entre le réservoir et la pompe.

7. Procédé selon la revendication 3, caractérisé en ce qu'une montée rapide de la valeur de pic est interprétée comme indiquant une augmentation de la quantité de liquide distribué pendant un cycle de la pompe.

8. Procédé selon la revendication 3, caractérisé en ce qu'une chute rapide de la valeur de pic est interprétée comme indiquant une diminution de la quantité de liquide administrée pendant un cycle de la pompe.

9. Procédé selon la revendication 3, caractérisé en ce qu'une chute rapide du niveau zéro est interprétée comme indiquant l'influence d'une pression hydrostatique négative externe.

10. Procédé selon la revendication 3, caractérisé en ce qu'une montée rapide du niveau zéro est interprétée comme indiquant l'influence d'une pression hydrostatique positive externe.

11. Procédé selon la revendication 3, caractérisé en ce qu'une montée rapide du niveau zéro est interprétée comme indiquant l'existence d'une obturation du cathéter.

12. Procédé selon la revendication 3, caractérisé en ce qu'une montée rapide du niveau zéro est interprétée comme indiquant l'existence de fissures sur le cathéter ou un débranchement du cathéter du corps du patient.

13. Dispositif pour la surveillance en continu d'un système d'administration en particulier pour l'utilisation dans l'administration d'insuline, constitué par une pompe volumétrique destinée à administrer une quantité prédéterminée de liquide pour chaque cycle de la pompe, une unité de commande de perfusion, une unité de mesure électronique et un cathéter de sortie muni d'un élément de mesure (1) selon l'une quelconque des revendications 1-12, caractérisé en ce qu'il comprend un élément de mesure (1) produisant un signal réfléchissant la pression réelle du cathéter et un système d'analyse (5) qui compare les valeurs mesurées aux valeurs enregistrées dans une mémoire du système d'analyse (5) et émet un signal lorsque les valeurs mesurées s'écartent des valeurs enregistrées dans la mémoire.